# EUROPEAN PATENT SPECIFICATION

(11) **EP 1 077 652 B1**
(45) Date of publication and mention of the grant of the patent: **20.08.2003**
(21) Application number: 98930681.6
(22) Date of filing: 12.05.1998
(51) Int. Cl.: A61B 17/80, A61B 17/04

(54) **BONE AUGMENTATION DEVICE**
KNOCHENVERSTÄRKUNGSEINRICHTUNG
DISPOSITIF D'AUGMENTATION OSSEUSE

(43) Date of publication of application: 28.02.2001
(73) Proprietor: SYNTHES AG CHUR, 7002 Chur (CH)
(72) Inventor: CURTIS, Raymond, CH-7260 Davos Dorf (CH); HEHLI, Markus, CH-7276 Frauenkirch (CH)
(74) Representative: Lusuardi, Werther Giovanni, Dr.
(86) International application number: PCT/EP98/02761
(87) International publication number: WO 99/058073

(56) References cited:
- EP-A- 0 520 177
- WO-A-93/19678
- WO-A-96/04852
- DE-A- 3 211 682
- FR-A- 1 550 029
- FR-A- 2 422 386
- US-A- 5 306 301

## Description

This invention concerns a bone augmentation device in accordance with the pre-characterising portion of Claim 1. A device as defined in the preamble of claim 1 in disclosed in EP-A-0 520 177.

Such bone augmentation devices are used for attachment of soft tissues to bone by means of commonly used sutures. Attachment of soft tissue to bone is a technique frequently required in orthopaedic surgical procedures. It is used in repair of soft tissue avulsions from bone as well as in reconstructive procedures. Augmentation devices are used in particular for attachment of avulsed tendons, ligaments and joint capsules to bone.

They are indicated for the following fields of application:

### A) shoulder instability and rotator cuff tears

The shoulder is dislocated more frequently than any other human joint causing limitation of motion and pain in athletes and nonathletes of all ages.

### B) knee instability

### C) tenodosis and ligamentous repair of the foot, ankle and wrist

One state of the art procedure of soft tissue attachment to bone is the classic Bankart procedure which is a widely accepted method of treating anterior-inferior gleno-humeral instability of the shoulder. It uses sutures that are inserted directly through transosseous tunnels. Although the surgical exposure involves minimal trauma and skin incision and leads to excellent clinical results with reported recurrence rate of only 3,5 to 4,0 % , the procedure of reattaching the torn ligament or tendon can be time consuming and difficult. While modifications that decrease the operating time for standard rotator cuff and Bankart lesion repair are available, these approaches are technically demanding.

Another known method for soft tissue attachment to bone is the use of surgical staples which, however, have a tendency to cut through the bone and tendon.

The invention as claimed aims at solving the above described problems.

The present invention provides a bone augmentation device as defined in Claim 1. The device according to the invention prevents the suture from cutting through the bone and prevents gapping between the soft tissue (tendon) and bone which would result in a poor healing. The consequence is that a stable repair will facilitate healing.

The device according to the invention consists of a perforated curved plate having a curvature R_{PL} in the range of 25 to 100 mm. For application to the humerus the curvature R_{PL} should be in the range of 35 to 70 mm, preferably in the range of 45 to 55 mm.

In order to prevent damage of the suture at the edges of the plate the edges at the outer periphery of the plate are rounded with a curvature R_{EP} in the range of 0,2 to 0,7 mm and the edges of the through holes are rounded with a curvature R_{EH} in the range of 0,2 to 0,8 mm.
The curvature R_{EP} of the peripheral edges purposefully is chosen in the range of 0,25 to 0,50 mm and preferably in the range of 0,3 to 0,4 mm. The curvature R_{EH} of the edges of the through holes purposefully is chosen in the range of 0,30 to 0,50 mm and preferably in the range of 0,35 to 0,45 mm.

The maximum thickness of the plate is below 1 mm and preferably below 0,75 mm. The maximum convex surface area F of the plate - including the area of the through holes - should be in the range of 100 - 250 mm², preferably in the range of 125 - 175 mm².

The device according to the invention needs at least one through hole and preferably at least two through holes. For many applications 4 to 7 through holes are ideal. The through holes should have a diameter in the range of 1,70 to 2,00 mm and preferably in the range of 1,80 to 1,90 mm. The minimum distance between two centres of the through holes is in the range of 3,0 to 4,3 mm and preferably in the range of 3,4 to 3,8 mm.

The device may be made of any recognised implant material but titanium is preferred because it can withstand higher loads compared to plastic materials. Alternatively titanium offers the possiblity to design plates with reduced height.

The various features of novelty which characterize the invention are pointed out with particularity in the claims annexed to and forming part of this disclosure. For the better understanding of the invention, its operating advantages and specific objects attained by its use, reference should be had to the accompanying drawings, examples and descriptive matter in which are illustrated and described preferred embodiments of the invention.

In the drawings:
- Fig. 1: is a top view of the device according to the invention;
- Fig. 2: is a section along line II-II of Fig. 1; and
- Fig. 3: is a section along line III-III of Fig. 1.

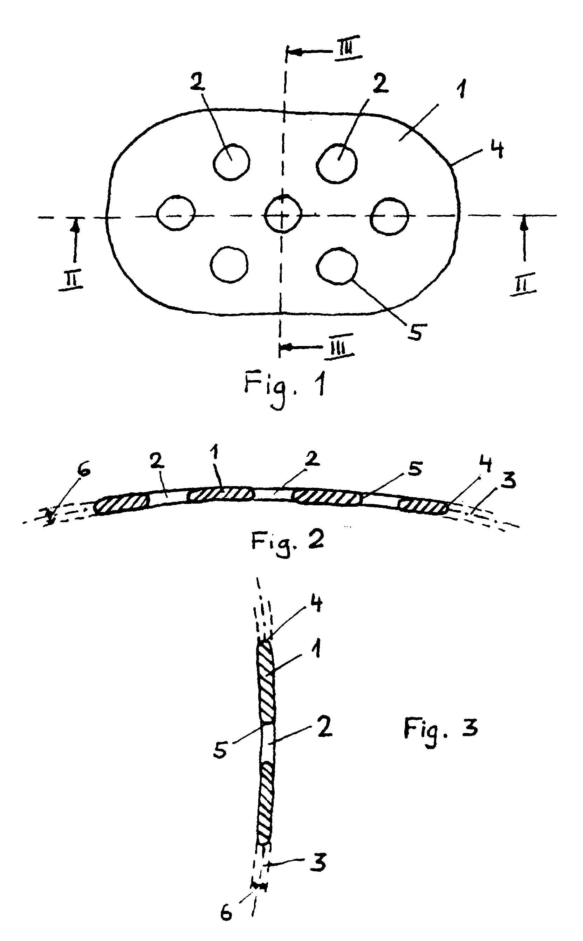

Figures 1 to 3 show a bone augmentation device for attachment of soft tissues to bone by means of sutures consisting of a curved plate 1 wholly made of titanium with a thickness of 0,7 mm. The plate 1 is provided with seven through holes 2 with a diameter of 1,85 mm and whose centres are regularly spaced at 3,6 mm from each other.

The plate 1 lies in a curved plane 3 having a curvature R_{PL} of 50 mm. The outer edges 4 of the plate 1 are rounded with a curvature R_{EP} of 0,35 mm and the edges 5 of the through holes 2 are also rounded with a curvature R_{EH} of 0,4 mm.
The plate 1 has a maximum convex surface area F - including the area of the through holes 2 - of approximately 150 mm².

Following is a short description of the method of operation with the device according to the invention.
First the tendon attachment site is selected by the surgeon. Using a burr, a trough which will later receive the tendon stump is selected. Holes are created in the bone at the desired attachment site. Suture stitches are created in the tendon, the end of the sutures are passed through the holes in the bone. The sutures are passed through the appropriate holes of the augmentation device. As the knots are tied the avulsed tendon and augmentation device are pulled onto the bone. the resulting repair is very stable and does not allow gapping between the end of the tendon and bone.

While the foregoing description and drawings represent the preferred embodiments of the present invention, it will be obvious for those skilled in the art that various changes and modifications may be made therein without departing from the scope of the present invention.

## Claims

1. Bone augmentation device for attachment of soft tissues to bone by means of sutures, comprising a curved bone plate (1) with rounded edges (4) and with at least one through hole (2), **characterized in that**
A) the bone plate (1) lies in a curved plane (3) having a curvature R_{PL} in the range of 25 to 100 mm;
B) the rounded edges (4) of the plate (1) have a curvature R_{EP} in the range of 0,2 to 0,7 mm;
C) the edges (5) of the through holes (2) are rounded with a curvature R_{EH} in the range of 0,2 to 0,8 mm; and
D) the bone plate (1) is made of a non-resorbable implant material.

2. Device according to claim 1, **characterized in that** the curvature R_{PL} of plane (3) is in the range of 35 to 70 mm, preferably in the range of 45 to 55 mm.

3. Device according to claim 1 or 2, **characterized in that** the curvature R_{EP} of the edges (4) is in the range of 0,25 to 0,50 mm, preferably in the range of 0,3 to 0,4 mm.

4. Device according to one of the claims 1 to 3, **characterized in that** the curvature R_{EH} of the edges (5) is in the range of 0,30 to 0,50 mm, preferably in the range of 0,35 to 0,45 mm.

5. Device according to one of the claims 1 to 4, **characterized in that** the maximum thickness (6) of the plate is below 1 mm, preferably below 0,75 mm.

6. Device according to one of the claims 1 to 6, **characterized in that** the plate (1) has a maximum convex surface area F including the area of the through holes (2) in the range of 100 - 250 mm², preferably in the range of 125 - 175 mm².

7. Device according to one of the claims 1 to 6, **characterized in that** the plate (1) has at least two through holes (2) and preferably 4 to 7 through holes (2).

8. Device according to one of the claims 1 to 7, **characterized in that** the through holes (2) have a diameter in the range of 1,70 to 2,00 mm, preferably in the range of 1,80 to 1,90 mm.

9. Device according to one of the claims 1 to 8, **characterized in that** the minimum distance between two centres of the through holes (2) is in the range of 3,0 - 4,3 mm, preferably in the range of 3,4 to 3,8 mm.

10. Device according to one of the claims 1 to 9, **characterized in that** it is made wholly from titanium.

## Patentansprüche

1. Knochenaufbauvorrichtung zur Befestigung von Weichteilen an Knochen mit Hilfe von Nähten, welche eine gekrümmte Knochenplatte (1) mit abgerundeten Kanten (4) und mit zumindest einer Durchgangsbohrung (2) umfasst,
**dadurch gekennzeichnet, dass**
A) die Knochenplatte (1) in einer gekrümmten Ebene (3) liegt, welche eine Krümmung R_{PL} im Bereich von 25 bis 100 mm aufweist;
B) die abgerundeten Kanten (4) der Platte (1) eine im Bereich von 0,2 bis 0,7 mm liegende Krümmung R_{EP} aufweisen;
C) die Kanten (5) der Durchgangsbohrungen (2) durch eine im Bereich von 0,2 bis 0,8 mm liegende Krümmung R_{EH} abgerundet sind; und
D) die Knochenplatte (1) aus einem nicht-resorbierbaren Implantatmaterial gefertigt ist.

2. Vorrichtung nach Anspruch 1, **dadurch gekennzeichnet, dass** die Krümmung R_{PL} der Ebene (3) im Bereich von 35 bis 70 mm, vorzugsweise im Bereich von 45 bis 55 mm gelegen ist.

3. Vorrichtung nach Anspruch 1 oder 2, **dadurch gekennzeichnet, dass** die Krümmung R_{EP} der Kanten (4) im Bereich von 0,25 bis 0,50 mm, vorzugsweise im Bereich von 0,3 bis 0,4 mm gelegen ist.

4. Vorrichtung nach einem der Ansprüche 1 bis 3, **dadurch gekennzeichnet, dass** die Krümmung R_{EH} der Kanten (5) im Bereich von 0,30 bis 0,50 mm, vorzugsweise im Bereich von 0,35 bis 0,45 mm gelegen ist.

5. Vorrichtung nach einem der Ansprüche 1 bis 4, **dadurch gekennzeichnet, dass** die maximale Dicke (6) der Platte weniger als 1 mm, vorzugsweise weniger als 0,75 mm beträgt.

6. Vorrichtung nach einem der Ansprüche 1 bis 5, **dadurch gekennzeichnet, dass** die Platte (1) einen die Zone mit den Durchgangsbohrungen (2) einschliessenden, maximalen konvexen Oberflächenbereich F im Bereich von 100 - 250 mm², vorzugsweise im Bereich von 125 - 175 mm² aufweist.

7. Vorrichtung nach einem der Ansprüche 1 bis 6, **dadurch gekennzeichnet, dass** die Platte (1) zumindest zwei Durchgangsbohrungen (2), vorzugsweise jedoch 4 bis 7 Durchgangsbohrungen (2) aufweist.

8. Vorrichtung nach einem der Ansprüche 1 bis 7, **dadurch gekennzeichnet, dass** die Durchgangsbohrungen (2) einen Durchmesser im Bereich von 1,70 bis 2,00 mm, vorzugsweise im Bereich von 1,80 bis 1,90 mm aufweisen.

9. Vorrichtung nach einem der Ansprüche 1 bis 8, **dadurch gekennzeichnet, dass** der Mindestabstand zwischen je zwei Mittelpunkten der Durchgangsbohrungen (2) im Bereich von 3,0 bis 4,3 mm, vorzugsweise im Bereich von 3,4 bis 3,8 mm gelegen ist.

10. Vorrichtung nach einem der Ansprüche 1 bis 9, **dadurch gekennzeichnet, dass** sie zur Gänze aus Titan gefertigt ist.

## Revendications

1. Dispositif d'ostéosynthèse pour la fixation de tissus mous à l'os au moyen de sutures, qui comprend une plaque osseuse incurvée (1) à bords (4) arrondis et au moins une perforation (2), **caractérisé en ce que**
A) la plaque osseuse (1) est située dans un plan incurvé (3) dont la courbure R_{PL} est comprise dans la plage de 25 à 100 mm;
B) les bords arrondis (4) de la plaque (1) ont une courbure R_{EP} comprise dans la plage de 0,2 à 0,7 mm;
C) les bords (5) des perforations (2) sont arrondis à une courbure R_{EH} comprise dans la plage de 0,2 à 0,8 mm; et
D) la plaque pour os (1) est réalisée en un matériau d'implant non résorbable.

2. Dispositif selon la revendication 1, **caractérisé en ce que** la courbure R_{PL} du plan (3) est comprise dans la plage de 35 à 70 mm et de préférence dans la plage de 45 à 55 mm.

3. Dispositif selon la revendication 1 ou 2, **caractérisé en ce que** la courbure R_{EP} des bords (4) est comprise dans la plage de 0,25 à 0,50 mm, de préférence dans la plage de 0,3 à 0,4 mm.

4. Dispositif selon l'une des revendications 1 à 3, **caractérisé en ce que** la courbure R_{EH} des bords (5) est comprise dans la plage de 0,30 à 0,50 mm, de préférence dans la plage de 0,35 à 0,45 mm.

5. Dispositif selon l'une des revendications 1 à 4, **caractérisé en ce que** l'épaisseur maximale (6) de la plaque est inférieure à 1 mm et de préférence inférieure à 0,75 mm.

6. Dispositif selon l'une des revendications 1 à 6, **caractérisé en ce que** la plaque (1) présente une superficie maximale F de sa surface convexe, y compris la superficie des perforations (2), qui est comprise dans la plage de 100 à 250mm² et de préférence dans la plage de 125 à 175 mm².

7. Dispositif selon l'une des revendications 1 à 6, **caractérisé en ce que** la plaque (1) présente au moins deux perforations (2) et de préférence de 4 à 7 perforations (2).

8. Dispositif selon l'une des revendications 1 à 7, **caractérisé en ce que** les perforations (2) ont un diamètre compris dans la plage de 1,70 à 2,00 mm, de préférence dans la plage de 1,80 à 1,90 mm.

9. Dispositif selon l'une des revendications 1 à 8, **caractérisé en ce que** la distance minimale entre deux centres de perforations (2) est comprise dans la plage de 3,0 à 4,3 mm, et de préférence dans la plage de 3,4 à 3,8 mm.

10. Dispositif selon l'une des revendications 1 à 9, **caractérisé en ce qu'**il est entièrement réalisé en titane.
